(19) Europäisches Patentamt
European Patent Office
Office européen des brevets

(11) Publication number: **0 492 902 A1**

# (12) EUROPEAN PATENT APPLICATION

(21) Application number: **91311528.3**

(22) Date of filing: **11.12.91**

(51) Int. Cl.$^5$: **A61K 31/44,** A61K 31/55, C07D 453/02, C07D 453/06, C07D 487/08, C07D 471/08, C07D 221/22, // (C07D487/08, 209:00, 209:00), (C07D471/08, 221:00, 209:00)

(30) Priority: **21.12.90 GB 9027824**
**07.06.91 GB 9112307**

(43) Date of publication of application:
**01.07.92 Bulletin 92/27**

(84) Designated Contracting States:
**CH DE FR GB IT LI NL**

(71) Applicant: **MERCK SHARP & DOHME LTD.**
**Hertford Road**
**Hoddesdon Hertfordshire EN11 9BU (GB)**

(72) Inventor: **Lotti, Victor**
**214 Brookside Circle**
**Harleysville, Pennsylvania 19438 (US)**
Inventor: **Showell, Graham A.**
**5 Beehive Lane**
**Welwyn Garden City, Hertfordshire (GB)**

(74) Representative: **Thompson, John Dr. et al**
**Merck & Co., Inc. European Patent**
**Department Terlings Park Eastwick Road**
**Harlow, Essex CM20 2QR (GB)**

(54) **Substituted pyridine derivatives for use in the treatment of glaucoma.**

(57) The use of a compound of structural formula (I):

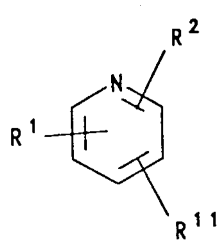

( I )

or a pharmaceutically acceptable salt thereof or a prodrug thereof; wherein

$R^1$ represents a non-aromatic azabicyclic ring system having more than 5 ring atoms selected from:

EP 0 492 902 A1

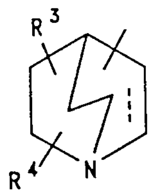
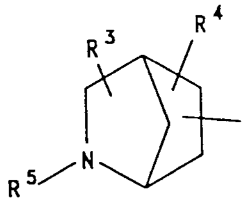
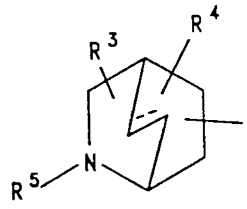
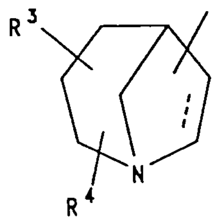
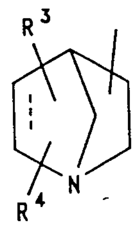

wherein the broken line represents an optional chemical bond ;

the substituents $R^3$ and $R^4$ may be present at any position, including the point of attachment to the pyridine ring, and independently represent hydrogen, $C_{1-4}$ alkyl, halo, $C_{1-4}$ alkoxy, hydroxy, carboxy or $C_{1-4}$ alkoxycarbonyl ; or $R^3$ and $R^4$ together represent carbonyl ; and

$R^5$represents hydrogen or $C_{1-4}$ alkyl ;

provided that $R^1$ is attached to the pyridine ring other than at the 2'-carbon position of $R^1$ ; and

$R^2$ and $R^{11}$ independently represent hydrogen, halo, $-CF_3$, $-OR^6$, $-NR^6R^7$, -CN, $-COR^8$, $C_{1-8}$ alkyl or $C_{2-8}$ alkenyl ; wherein $R^6$ is hydrogen or $C_{1-6}$ alkyl ; $R^7$ is hydrogen or $C_{1-6}$ alkyl ; and $R^8$ represents $-OR^6$ or $-NR^6R^7$ ; other than 3-hydroxy-3-(2-pyridinyl)-1-azabicyclo[2.2.2]-octane and 3-(2-pyridinyl)-1-azabicyclo[2.2.2]oct-2-ene ;

for the manufacture of a medicament for treating glaucoma and/or for reducing intraocular pressure.

The present invention relates to a class of substituted pyridine compounds for use in the treatment of glaucoma, formulations containing them and their synthesis.

Glaucoma is an ocular disorder associated with elevated intraocular pressures which are too high for normal function and may result in irreversible loss of visual function. If untreated, glaucoma may eventually lead to blindness. Ocular hypertension, i.e. the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Topical administration to the eye of agents such as pilocarpine may be used in order to improve the outflow of aqueous humour and reduce the intraocular pressure (see Drugs, 1979, vol. 17, 38; and Drugs and Ther. Bull., 1989, vol 21, 85), but these have associated side effects such as emesis and myosis.

We have now found compounds which lower the intraocular pressure without exhibiting side effects to the extent associated with hitherto-known drugs used against glaucoma which act through cholinergic mechanisms.

In J. Med. Chem. (1971), 14 (6), 554-556 are disclosed 3-hydroxy-3-(2-pyridinyl)-1-azabicyclo[2.2.2]octane and 3-(2-pyridinyl)-1-azabicyclo[2.2.2]oct-2-ene as intermediates in the preparation of compounds evaluated for their nicotinic activity. The former compound was itself tested and found in some tests to perform as a weak nicotinic agonist, but in other tests not to perform as a nicotinic agonist. It has now been found that a class of pyridines, having a broader range of substituents, effect reduction of intraocular pressure without exhibiting severe adverse effects on pupil size. Some of these compounds also exhibit nicotinic activity.

The compounds for use according to the present invention may be represented by structural formula (I):

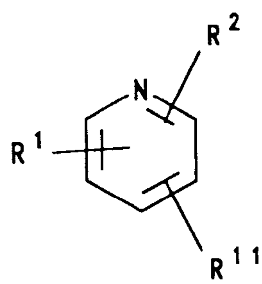

( I )

or a salt or prodrug thereof; wherein

$R^1$ represents a non-aromatic azabicyclic ring system having more than 5 ring atoms selected from:

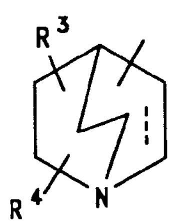

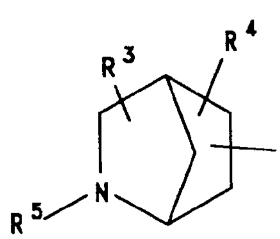

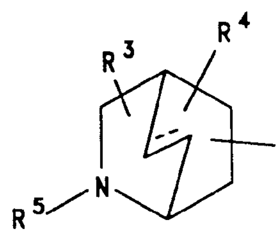

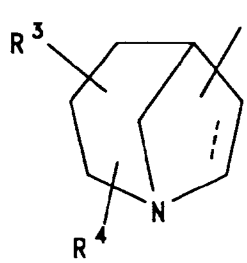

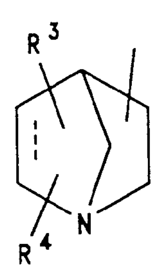

wherein the broken line represents an optional chemical bond;

the substituents $R^3$ and $R^4$ may be present at any position, including the point of attachment to the pyridine ring, and independently represent hydrogen, $C_{1-4}$ alkyl, halo, $C_{1-4}$ alkoxy, hydroxy, carboxy or $C_{1-4}$ alkoxycarbonyl; or $R^3$ and $R^4$ together represent carbonyl; and

$R^5$ represents hydrogen or $C_{1-4}$ alkyl;

provided that $R^1$ is attached to the pyridine ring other than at the 2′-carbon position of $R^1$; and

$R^2$ and $R^{11}$ independently represent hydrogen, halo, $-CF_3$, $-OR^6$, $-NR^6R^7$, -CN, $-COR^8$, $C_{1-8}$ alkyl or $C_{2-8}$ alkenyl; wherein $R^6$ is hydrogen or $C_{1-6}$ alkyl; $R^7$ is hydrogen or $C_{1-6}$ alkyl; and $R^8$ represents $-OR^6$ or $-NR^6R^7$;

other than 3-hydroxy-3-(2-pyridinyl)-1-azabicyclo[2.2.2]octane and 3-(2-pyridinyl)-1-azabicyclo[2.2.2]oct-2-ene.

In the definition of $R^1$, it will be appreciated that the nitrogen atom in the azabicyclic ring system will carry a lone pair of electrons.

In the definition of $R^2$ and/or $R^{11}$, unless otherwise specified, "alkyl" and "alkenyl" groups may be straight, branched or cyclic groups.

One subclass of compounds for use according to the present invention is represented by formula (II):

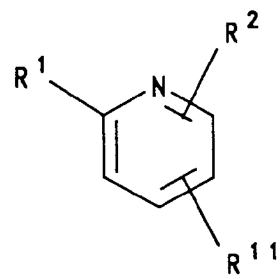

(II)

wherein $R^1$, $R^2$ and $R^{11}$ are as defined above.

Suitably, $R^1$ is azanorbornane, quinuclidine, isoquinuclidine, azabicyclo[2.2.2]octene or 1-azabicyclo[3.2.1]octane, any of which may be either unsubstituted or substituted with methyl, hydroxy, fluoro, chloro or methoxycarbonyl. Preferably, $R^1$ is quinuclidine, 1-azabicyclo[2.2.1]heptane or isoquinuclidine (especially quinuclidine) optionally substituted with methoxycarbonyl. Preferably, the azabicyclic ring is bonded at the 2- or 3-position of the pyridine ring, especially the 2-position.

Preferably the groups $R^2$ and/or $R^{11}$ independently represent hydrogen, halo, $-CF_3$, $-OR^6$, $-NR^6R^7$, -CN, $-COR^8$, $C_{1-6}$ alkyl, $C_{3-6}$ cycloalkyl or $C_{2-6}$ alkenyl; especially where $R^6$ and $R^7$ are each independently hydrogen or methyl. Particular values of the groups $R^2$ and/or $R^{11}$ are hydrogen, chloro, methyl, ethyl, isopropyl, cyclopropyl, methoxy, ethoxy, isopropoxy, n-butoxy, methoxycarbonyl and ethoxycarbonyl Preferred values are methyl, methoxy, ethoxy, chloro and hydrogen, especially chloro.

Suitably, the group $R^3$ is hydrogen or methyl; and $R^4$ is hydrogen, $C_{1-4}$ alkoxy, $C_{1-4}$ alkyl, $C_{1-4}$ alkoxycarbonyl, halo or hydroxy, preferably hydrogen, methoxy, methyl, fluoro, chloro, hydroxy or methoxycarbonyl. Preferably one or both of $R^3$ and $R^4$ is hydrogen. More preferably, $R^3$ is hydrogen and $R^4$ is hydrogen, hydroxy or methoxycarbonyl, especially hydrogen.

Preferably, the group $R^5$ represents hydrogen or methyl.

A particularly preferred subgroup of formula (I) is wherein:

$R^1$ is a ring system shown above attached to the pyridine ring at the 3′-carbon position of $R^1$;

$R^2$ is hydrogen, halo (especially chloro), $-OR^6$ (especially methoxy or ethoxy), $-NR^6R^7$ (especially dimethylamino) or $C_{1-6}$ alkyl (especially methyl); and $R^{11}$ is hydrogen;

other than 3-hydroxy-3-(2-pyridinyl)-1-azabicyclo[2.2.2]octane and 3-(2-pyridinyl)-1-azabicyclo[2.2.2]oct-2-ene.

Especially preferred is when the azabicycle ($R^1$) is either unsubstituted or substituted at the 3′-position by hydrogen, halo (particularly chloro) or hydroxy, provided that, when $R^1$ is quinuclidine and $R^2$ and $R^{11}$ are each hydrogen, then the substituent ($R^3$ or $R^4$) is other than 3′-hydroxy.

Examples of suitable compounds for use according to the present invention include those of structural formula (IA) or salts or prodrugs thereof:

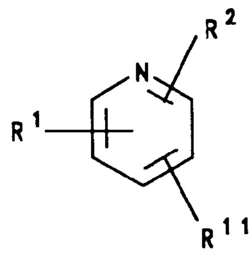

(IA)

wherein

$R^1$ is selected from

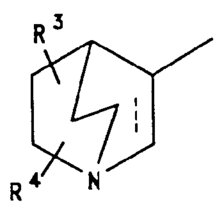

,

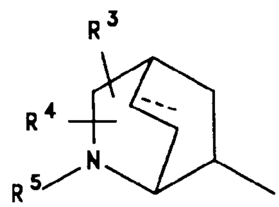

and

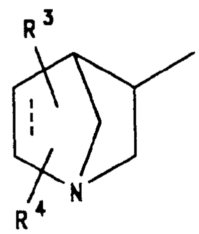

;

$R^2$ and $R^{11}$ are independently selected from hydrogen, halo, $-OR^6$, $-NR^6R^7$ and $C_{1-6}$ alkyl; and

$R^3$ and $R^4$ are independently selected from hydrogen, halo and hydroxy;

other than 3-hydroxy-3-(2-pyridinyl)-1-azabicyclo[2.2.2]octane and 3-(2-pyridinyl)-1-azabicyclo[2.2.2]oct-2-ene.

For example, $R^1$ may be 1-azabicyclo[2.2.1]heptane or quinuclidine;

$R^2$ may be halo such as chloro or $-OR^6$ such as methoxy or ethoxy; and

$R^3$, $R^4$, $R^5$ and $R^{11}$ may each be hydrogen.

One group of prodrugs of compounds of use in this invention have a substituent on the pyridine ring which is hydrolysable in vivo to an amino group.

Groups which are hydrolysable in vivo to an amino group on the compounds of use in this invention may be readily ascertained by administering the compound to a human or animal and detecting, by conventional analytical techniques, the presence of the corresponding compound having an amino substituent in the urine of a human or animal. Examples of such groups include, for example, amido and urethane substituents, in particular a group of formula -NH.Q, wherein Q represents CHO, COR or $CO_2R$, and R represents an optionally substituted hydrocarbon group.

In this context, the hydrocarbon group R includes groups having up to 20 carbon atoms, suitably up to 10 carbon atoms, conveniently up to 6 carbon atoms. Suitable groups R include $C_{1-9}$ alkyl, $C_{2-6}$ alkenyl, $C_{2-6}$ alkynyl, $C_{3-7}$ cycloalkyl, $C_{3-7}$ cycloalkyl($C_{1-6}$)alkyl, aryl, and aryl($C_{1-6}$)alkyl. The alkyl group R may be straight or branched chain and may contain, for example, up to 12 carbon atoms, suitably from 1 to 6 carbon atoms. In particular the group may be substituted methyl, ethyl, n- or iso-propyl, n-, sec-, iso- or tert-butyl, n- or iso-heptyl, or n- or iso-octyl. Suitable cycloalkyl groups include cyclopentyl and cyclohexyl. The aryl group R includes phenyl and naphthyl optionally substituted with up to five, preferably up to three, substituent groups.

Most of the compounds for use according to this invention have at least one asymmetric centre and often more than one; and can therefore exist as both enantiomers and diastereoisomers. In particular, those compounds possessing an unsymmetrical azabicyclic ring system may exist as exo and endo diastereoisomers. It is to be understood that the invention covers the use of all such isomers and mixtures thereof.

Also included within the scope of the present invention is the use of salts of these compounds. It will be appreciated that salts of the compounds for use in medicine will be non-toxic pharmaceutically acceptable salts. Other salts may, however, be useful in the preparation of the compounds of use in the invention or their non-toxic pharmaceutically acceptable salts. Acid addition salts, for example, may be formed by mixing a solution of the compound with a solution of a pharmaceutically acceptable non-toxic acid such as hydrochloric acid, fumaric acid, oxalic acid, maleic acid, succinic acid, acetic acid, citric acid, tartaric acid, p-toluenesulphonic acid, carbonic acid or phosphoric acid. Preferred are the hydrochloride, hydrogen oxalate, maleate, tartrate and tosylate

salts. Where the compound carries a carboxylic acid group the invention also contemplates salts thereof, preferably non-toxic pharmaceutically acceptable salts thereof, such as the sodium, potassium and calcium salts thereof.

Salts of amine groups may also comprise the quaternary ammonium salts in which the amino nitrogen atom carries an alkyl, alkenyl, alkynyl or aralkyl group.

Examples of compounds for use according to this invention include:
endo-3-[2-(6-methoxypyridin)yl]-1-azabicyclo[2.2.1]heptane;
3-[2-(6-ethoxypyridin)yl]-1-azabicyclo[2.2.2]octane;
3-[2-(6-methoxypyridin)yl]-1-azabicyclo[2.2.2]octane;
3-[2-(6-chloropyridin)yl]-1-azabicyclo[2.2.2]octane;
and salts and prodrugs thereof.

The compounds for use according to this invention may be prepared as described hereinbelow or by processes analogous thereto known to persons skilled in the art.

The compounds for use according to this invention wherein $R^3$ and $R^4$ are each other than hydroxy or carboxy substituted on the azabicycle at the point of attachment to the pyridine ring may be prepared by a process which comprises the dehydroxylation or decarboxylation of a compound of formula (III) [which is a sub-class of the compounds of formula (I)] or a salt thereof:

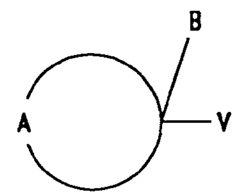

(III)

wherein V represents a pyridine ring substituted by the groups $R^2$ and $R^{11}$ as defined above; A represents the residue of an azacyclic or azabicyclic ring as defined above for $R^1$; and B represents hydroxy or a carboxycontaining group.

When the group B in compound (III) is hydroxy, it may be removed by chlorination and elimination, followed by hydrogenation. For example, chlorination and elimination may be effected by treatment with phosphorus oxychloride in the presence of triethylamine, or with thionyl chloride followed, where necessary, by DBN. The chloride or the unsaturated product may then be hydrogenated under conventional conditions, such as over 10% palladium/carbon in methanol. Alternatively, the compound (III) may be dehydroxylated by pyrolysis of the xanthate ester followed by hydrogenation. The xanthate ester may be formed by deprotonation of (III) with a metal hydride followed by quenching with carbon disulphide and subsequent alkylation. Alternatively, the compound (III) may be dehydroxylated by the use of thionyl chloride followed by treatment with tributyltin hydride in a solvent such as tetrahydrofuran in the presence of a radical initiator such as azabisisobutyronitrile.

By analogy with the disclosure in J. Med. Chem. (1971), 14, 554-6, the compounds of formula (III) wherein B is hydroxy may be prepared by reaction of a ketone of formula (IV) with a metal derivative of a pyridine of formula (V):

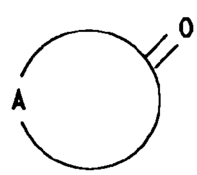

(IV)

M - V

(V)

wherein A and V are as defined above; and M represents a metal atom, for example lithium. The metal derivative, for instance, may be prepared by reacting the corresponding halo-substituted pyridine such as iodo- or

bromo-substituted pyridine with t-butyllithium, n-butyllithium or the metal alone.

When the group B in compound (III) is carboxy, it may be removed by standard decarboxylation techniques such as by heating in aqueous solution made to pH1 with hydrochloric acid.

The compounds of formula (III) wherein B represents a carboxy-containing group may be prepared by reaction of a compound of formula (VI) with a compound of formula (VII):

$$R^1 - W \qquad\qquad Hal - V$$

(VI) (VII)

wherein $R^1$ and V are as defined above, Hal represents halo (such as chloro, bromo or fluoro), and W represents cyano, a carboxylic acid group or a derivative thereof which activates the adjacent position such as an alkyl ester; and subsequently, where necessary, converting the group W to carboxy, preferably by hydrolysis.

Preferably, W represents an alkyl ester group such as methoxycarbonyl. Preferably, the halo group is chloro or fluoro. The reaction between compounds (VI) and (VII) may be carried out in the presence of a strong base such as lithium diisopropylamide (which may be prepared in situ from n-butyllithium and diisopropylamine) in a solvent such as tetrahydrofuran.

The azabicyclic moiety may be introduced into the molecules concerned by methods known from the art, in particular by methods analogous to those described in EP-A-0239309.

After any of the above described processes is complete, one substituent can be converted to another. For example, an amino group may be converted to chloro, or hydrazo ($-NHNH_2$), via the intermediacy of diazonium ($-N_2$). Similarly, a chloro substituent may be converted to methoxy by reaction with a nucleophile such as methoxide, or to an alkyl group by reaction with a tetraalkyl stannane under palladium catalysis; alkoxycarbonyl groups may be converted, via carboxy, to an amino substituent ($-NH_2$); and methoxy may be converted to hydroxy by treatment with concentrated hydrobromic acid.

In any of the above reactions it may be necessary and/or desirable to protect any sensitive groups in the compounds. For example, if the reactants employed include amino, carboxy, keto, hydroxy or thiol groups, these may be protected in conventional manner. Thus, suitable protecting groups for hydroxy groups include silyl groups such as trimethylsilyl or t-butyl-dimethylsilyl, and etherifying groups such as tetrahydropyranyl; and for amino groups include benzyloxycarbonyl and t-butoxycarbonyl. Keto groups may be protected in the form of a ketal. Carboxy groups are preferably protected in a reduced form such as in the form of their corresponding protected alcohols, which may be subsequently oxidised to give the desired carboxy group. Thiol groups may be protected by disulphide formation, either with the thiol itself or with another thiol to form a mixed disulphide. The protecting groups may be removed at any convenient stage in the synthesis of the desired compound according to conventional techniques.

When administered for the treatment of elevated intraocular pressure or glaucoma, the active compound is preferably administered topically to the eye, although systemic treatment is also possible. The dose administered can be from as little as 0.1 to 25 mg or more per day, singly, or preferably on a 2 to 4 dose per day regimen although a single dose per day is satisfactory.

The present invention therefore also provides a pharmaceutical formulation suitable for use in reducing intraocular pressure or for treating glaucoma which formulation comprises a compound of formula (I) and a pharmaceutically acceptable carrier.

It will be understood that any formulation may further comprise another active ingredient such as another antiglaucoma agent for example a topical carbonic anhydrase inhibitor.

When given systemically, the drug can be given by any route, although the oral route is preferred. In oral administration, the drug can be employed in any of the usual dosage forms such as tablets or capsules, either in a contemporaneous delivery or sustained release form. Any number of the usual excipients or tabletting aids can likewise be included.

When given by the topical route, the active compound or an ophthalmologically acceptable salt thereof such as the hydrochloride salt is formulated into an ophthalmic preparation. In such formulations, from 0.0005% to 15% by weight can be employed. The objective is to administer a dose of from 0.1 to 1.0 mg per eye per day to the patient, with treatment continuing so long as the condition persists.

Thus, in an ophthalmic solution, insert, ointment or suspension for topical delivery, or a tablet, intramuscular or intravenous composition for systemic delivery, the active medicament or an equivalent amount of a salt thereof is employed, the remainder being carrier, excipients, preservatives and the like as are customarily used in such compositions.

The active drugs of use in this invention are most suitably administered in the form of ophthalmic pharmaceutical compositions adapted for topical administration to the eye such as a suspension, ointment, or as a solid insert. A preferred composition is eye drops. Formulations of these compounds may contain from 0.0005 to 15% and especially 0.05% to 2% of medicament. Higher dosages as, for example, about 10% or lower dosages can be employed provided the dose is effective in reducing or controlling elevated intraocular pressure. As a unit dosage from between 0.001 to 10.0 mg, preferably 0.005 to 2.0 mg, and especially 0.1 to 1.0 mg of the compound is generally applied to the human eye, generally on a daily basis in single or divided doses so long as the condition being treated persists.

This invention therefore further provides a pharmaceutical formulation adapted for topical administration to the eye which formulation comprises a compound of formula (I) and a carrier suitable for topical administration.

These hereinbefore described dosage values are believed accurate for human patients and are based on the known and presently understood pharmacology of the compounds, and the action of other similar entities in the human eye. As with all medications, dosage requirements are variable and must be individualized on the basis of the disease and the response of the patient.

For topical administration, pharmaceutically acceptable carriers are, for example, water, mixtures of water and water-miscible solvents such as lower alkanols or arylalkanols, vegetable oils, polyalkylene glycols, petroleum based jelly, ethyl cellulose, ethyl oleate, carboxymethylcellulose, polyvinylpyrrolidone, isopropyl myristate and other conventionally-employed non-toxic, pharmaceutically acceptable organic and inorganic carriers. The pharmaceutical preparation may also contain non-toxic auxiliary substances such as emulsifying, preserving, wetting and bodying agents, for example polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenyl ethanol, buffering ingredients such as sodium chloride, sodium borate, sodium acetates, gluconate buffers, and other conventional ingredients such as sorbitan monolaurate, triethanolamine oleate, polyoxyethylene sorbitan monopalmitylate, dioctyl sodium sulfosuccinate, monothioglycerol, thiosorbitol and ethylenediamine tetraacetic acid. Additionally, suitable ophthalmic vehicles can be used as carrier media for the present purpose including conventional phosphate buffer vehicle systems, isotonic boric acid vehicles, isotonic sodium chloride vehicles and isotonic sodium borate vehicles.

The pharmaceutical formulation may also be in the form of a solid insert such as one which after dispensing the drug remains essentially intact, or a bioerodible insert that is soluble in lachrymal fluids or otherwise disintegrates.

The present invention will now be illustrated by the following Descriptions.

**DESCRIPTION A**

3-[2-(6-Ethoxypyridin)yl]-1-azabicyclo[2.2.2]octane Hydrogen Oxalate

Prepared as described in Example 26 of EP-A-0412798.

**DESCRIPTION B**

Endo-3-[2-(6-methoxypyridin)yl]-1-azabicyclo[2.2.1]heptane Hydrogen Oxalate

Prepared as described in Example 32 of EP-A-0412798.

**DESCRIPTION C**

3-[2-6-Methoxypyridin)yl]-1-azabicyclo[2.2.2]octane Dihydrochloride

Prepared as described in Example 2 of EP-A-0412798.

**DESCRIPTION D**

3-[2-(6-Chloropyridin)yl]-1-azabicyclo[2.2.2]octane Hydrogen Oxalate

Prepared as described in Example 1 of EP-A-0412798.

## BIOLOGICAL ACTIVITY

| Compound of | Concentration | Change in IOP[a] |
|---|---|---|
| Description A | 0.05% | -3.0 |
| Description B | 0.05% | -4.5 |
| Description C | 0.05% | -3.0 |
| Description D | 0.5% | -3.0 |

FOOTNOTE: a, Maximum mmHg change in intraocular pressure

## FORMULATIONS

The compounds of the Descriptions may be formulated as follows:

Eye Drops

| | |
|---|---|
| The pharmaceutically acceptable salt of the active compound | 0.5% |
| Benzalkonium chloride solution | 0.02% v/v |
| Disodium edetate | 0.05% |
| NaCl | 0.8% |
| Water for injections | to 100% |

The formulation is sterilised by autoclaving.

## Claims

1. The use of a compound of structural formula (I):

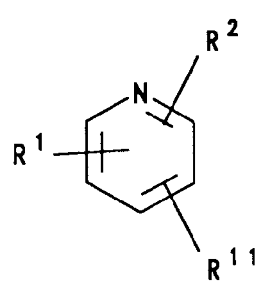

( I )

or a pharmaceutically acceptable salt thereof or a prodrug thereof; wherein

$R^1$ represents a non-aromatic azabicyclic ring system having more than 5 ring atoms selected from:

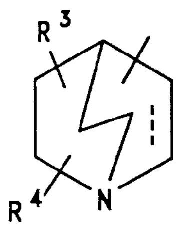

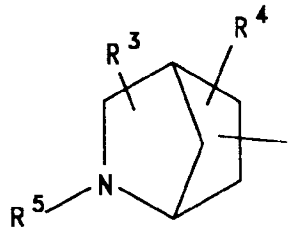

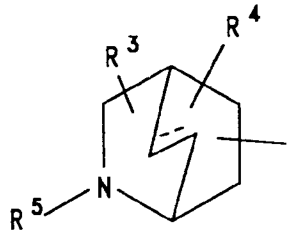

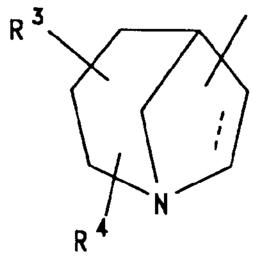

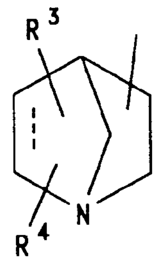

wherein the broken line represents an optional chemical bond;

the substituents $R^3$ and $R^4$ may be present at any position, including the point of attachment to the pyridine ring, and independently represent hydrogen, $C_{1-4}$ alkyl, halo, $C_{1-4}$ alkoxy, hydroxy, carboxy or $C_{1-4}$ alkoxycarbonyl; or $R^3$ and $R^4$ together represent carbonyl; and

$R^5$ represents hydrogen or $C_{1-4}$ alkyl; provided that $R^1$ is attached to the pyridine ring other than at the 2′-carbon position of $R^1$; and

$R^2$ and $R^{11}$ independently represent hydrogen, halo, $-CF_3$, $-OR^6$, $-NR^6R^7$, -CN, $-COR^8$, $C_{1-8}$ alkyl or $C_{2-8}$ alkenyl; wherein $R^6$ is hydrogen or $C_{1-6}$ alkyl; $R^7$ is hydrogen or $C_{1-6}$ alkyl; and $R^8$ represents $-OR^6$ or $-NR^6R^7$; other than 3-hydroxy-3-(2-pyridinyl)-1-azabicyclo[2.2.2]octane and 3-(2-pyridinyl)-1-azabicyclo[2.2.2]oct-2-ene; for the manufacture of a medicament for treating glaucoma and/or for reducing intraocular pressure.

2. The use as claimed in claim 1 of a compound of formula (II):

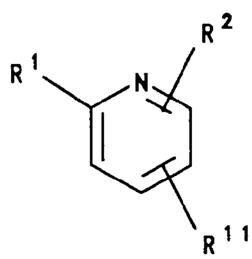

(II)

wherein $R^1$, $R^2$ and $R^{11}$ are as defined in claim 1.

3. The use as claimed in claim 1 or claim 2 wherein $R^1$ is quinuclidine, 1-azabicyclo[2.2.1]heptane or isoquinuclidine, optionally substituted with methoxycarbonyl.

4. The use as claimed in any one of the preceding claims wherein $R^2$ and $R^{11}$ independently represent hydrogen, chloro, methyl, ethyl, isopropyl, cyclopropyl, methoxy, ethoxy, isopropoxy, n-butoxy, methoxycarbonyl or ethoxycarbonyl.

5. The use as claimed in claim 1 wherein

$R^1$ is as defined in claim 1, attached to the pyridine nucleus at the 3′-carbon position of $R^1$;
$R^2$ is hydrogen, halo, $-OR^6$, $-NR^6R^7$ or $C_{1-6}$ alkyl, in which $R^6$ and $R^7$ are as defined in claim 1; and
$R^{11}$ is hydrogen;

other than 3-hydroxy-3-(2-pyridinyl)-1-azabicyclo[2.2.2]octane and 3-(2-pyridinyl)-1-azabicyclo[2.2.2]oct-2-ene.

**6.** The use as claimed in claim 1 of a compound of formula (IA) or a pharmaceutically acceptable salt thereof or a prodrug thereof:

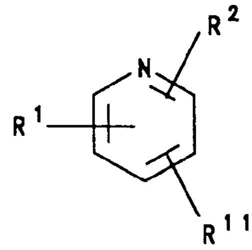

(IA)

wherein
$R^1$ is selected from

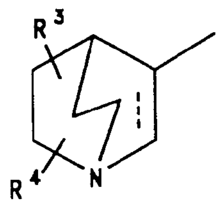
,
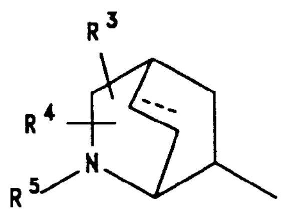
and
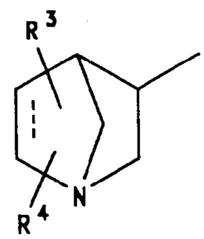
;

$R^2$ and $R^{11}$ are independently selected from hydrogen, halo, $-OR^6$, $-NR^6R^7$ and $C_{1-6}$ alkyl; and
$R^3$ and $R^4$ are independently selected from hydrogen, halo and hydroxy;

other than 3-hydroxy-3-(2-pyridinyl)-1-azabicyclo[2.2.2]octane and 3-(2-pyridinyl)-1-azabicyclo[2.2.2]oct-2-ene.

**7.** The use as claimed in claim 6 wherein
$R^1$ is 1-azabicyclo[2.2.1]heptane or quinuclidine;
$R^2$ is halo or $-OR^6$ in which $R^6$ is as defined in claim 1; and
$R^3$, $R^4$, $R^5$ and $R^{11}$ are all hydrogen.

**8.** The use as claimed in any one of the preceding claims of a compound selected from:
endo-3-[2-(6-methoxypyridin)yl]-1-azabicyclo[2.2.1]heptane;
3-[2-(6-ethoxypyridin)yl]-1-azabicyclo[2.2.2]octane;
3-[2-(6-methoxypyridin)yl]-1-azabicyclo[2.2.2]octane;
3-[2-(6-chloropyridin)yl]-1-azabicyclo[2.2.2]octane;
and pharmaceutically acceptable salts thereof and prodrugs thereof.

**9.** A pharmaceutical composition adapted for use in reducing intraocular pressure and/or for treating glaucoma which comprises a compound of formula (I) as defined in claim 1 or a pharmaceutically acceptable salt thereof or a prodrug thereof in association with a pharmaceutically acceptable carrier.

**10.** A pharmaceutical composition adapted for topical administration to the eye which comprises a compound of formula (I) as defined in claim 1 or a pharmaceutically acceptable salt thereof or a prodrug thereof in association with a carrier suitable for topical administration.

European Patent Office

# EUROPEAN SEARCH REPORT

Application Number

EP 91 31 1528

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 327 155 (MERCK SHARP & DOHME LTD)<br>* claims 1,2,7,9 *<br>--- | 1,9,10 | A61K31/44<br>A61K31/55<br>C07D453/02<br>C07D453/06<br>C07D487/08<br>C07D471/08<br>C07D221/22<br>//(C07D487/08, 209:00,209:00)<br>(C07D471/08, 221:00,209:00) |
| A | EP-A-0 370 415 (BOEHRINGER INGELHEIM KG)<br>* page 3, line 42 - line 48; claims 1,3-5 *<br>--- | 1,9,10 | |
| A,D | JOURNAL OF MEDICINAL CHEMISTRY<br>vol. 14, no. 6, 1971,<br>pages 554 - 557;<br>F.D. REED ET AL: 'Quinuclidine analogs of tobacco alkaloids'<br>* page 555, column 1, compounds 3a-b *<br>--- | 1 | |
| P,A,<br>D | EP-A-0 412 798 (MERCK SHARP & DOHME LTD)<br>* claims 1-9 *<br>----- | 1-10 | |

| TECHNICAL FIELDS SEARCHED (Int. Cl.5 ) |
|---|
| A61K<br>C07D |

The present search report has been drawn up for all claims

| Place of search | Date of completion of the search | Examiner |
|---|---|---|
| BERLIN | 18 MARCH 1992 | van Amsterdam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)